# EUROPÄISCHE PATENTANMELDUNG

(11) **EP 1 595 528 A1**
(43) Veröffentlichungstag der Anmeldung: **16.11.2005**
(21) Anmeldenummer: 03027652.1
(22) Anmeldetag: 14.05.2004
(51) Int. Cl.: A61K 7/48

(54) **Hyaferm-Komposite**

(71) Anmelder: Fibona Health Products GmbH, 65183 Wiesbaden (DE)
(72) Erfinder: Fleischer, Lutz-Günther, 10369 Berlin (DE); Gerber, Gerhard, Prof. Dr., 10319 Berlin (DE); Westphal, Günter, Prof. Dr., 10319 Berlin (DE); Lippert, Eberhard, Dr., 13156 Berlin (DE)
(74) Vertreter: Sammer, Dietrich

(57) **Zusammenfassung**

Die Hyaferm-Komposite sind topisch wirksame Basisprodukte, die aus Hyaferm und pflanzlichen mykotischen, mikobiellen und/oder synthetischen Polysacchariden mit spezifischen funktionellen Eigenschaften bestehen.

Hyaferm wird nach besonderen Verfahren aus Hyaluronsäure bzw. Hyaluronaten mikrbiellem oder tierischen Ursprungs bereitet.

Die Ansprüche zielen auf Polysaccharide vor allem nativen Ursprungs, die nichtkovatent an das Hyaferm binden, aus anomeren Saccharidbausteinen in der Furanoseoder/und Pyranoseform bestehen und einen differenten Verzweigungsgrad von 0-15 %, besonders 3-6 %, sowie einen gegebenen Proteinanteil von 15 % aufweisen.

Bevorzugt werden native oder modifizierte Verbindungen, die vor allem infolge ihres Molekulargewichts biochemische Vorgänge auf und in der Haut stabilisieren. Zudem müssen ausgewählten Polysaccaharidkomponenten in der Kombination mit dem Hyaferm mehreren funktionellen Ansprüchen genügen, die die beabsichtigte topische Wirksamkeit kosmetischer Basisprodukte gewährleisten. Zuzusetzen sind kombinierte Lösungsvermittler wie niedermolekulare Saccharide, Harnstoffe, mit Wasser mischbare, aber in der Polarität dem Wasser nachgeordnete Lösungsmittel bzw. Polyoxymethylene, - ethylene oder Emulgatoren wie Glycolipide, Partialglyceride bzw. Phosphatide.

## Beschreibung

Die Hyaferm-Komposite sind kosmetische Basisprodukte, die aus Hyaferm und pflanzlichen, mykotischen, mikrobiellen und/oder synthetischen Polysacchariden bestehen.
Hayferm steht für nach besonderem Verfahren bereitete Hyaluronsäure oder Hyaluronate mikrobiellen oder tierischen Ursprungs. Über kosmetisch wirksame Kombinationen aus Hyaluronsäure und Sacchariden ist schon berichtet worden. Allerdings wurden wie in DE 4439575 A1, JP A-5-508161, JP A-6-508169, US-P 4,636,524/ 4,582,865/ 5,266,563 und 5,087,446 die Kohlenhydrate meist in Gegenwart bifunktioneller Reagentien kovalent an die Hyaluronsäure gebunden. Dabei wird die transdermale Eigenschaft der Hyaluronsäure deutlich beeinträchtigt. Die Interaktionen der Saccharide mit der Hyaluronsäure vermittels nicht-kovalenter Bindungen wurden vor allem genutzt, um das Gelbildungsvermögen der Hyaluronsäure zu modifizieren. Die damit verbundenen Ansprüche sind u. a. in US-P 5,644,049 und CA 1140469 formuliert. Hyaluronsäure- und Hyaluronsäure-Saccharid-Gele werden auch insbesondere hergestellt, um DDS (drug delivery systems) aufzubauen, beschrieben in US-P 5,846,951/ 4,851,521/ 4,965,353 und EP 0251905, 0341745 sowie in R. V. Sparer u. a. "Control Release Delivery Systems" (hrsg. durch T. J. Roseman u.a.), Verlag Marcel Dekker Inc., New York 1983. Die nachfolgend formulierten Schutzansprüche zielen darauf ab, solche Kohlenhydrate, vor allem nativen Ursprungs, nicht-kovalent an das Hyaferm zu binden, die polymer sind und aus anomeren Saccharidbausteinen in der Furanose- oder/und Pyranoseform bestehen und einen differenten Verzweigungsgrad von 0 - 15 %, besonders 3 - 6 %, sowie einen gegebenen Proteinanteil von 15 % aufweisen.

Dabei werden solche Vertreter dieser nativen oder modifizierten Verbindungsklasse bevorzugt, die in Verbindung mit ihren Molekulargewichten biochemische Vorgänge auf und in der Haut stabilisieren. Zudem müssen die ausgewählten Polysaccharidkomponenten in der Kombination mit dem Hyaferm mehreren funktionellen Ansprüchen genügen, die die beabsichtigte topische Wirksamkeit der kosmetischen Basisprodukte gewährleisten. Von der Kombination Lösungsvermittler wie niedermolekulare Saccharide, Harnstoffe, mit Wasser mischbare, aber in der Polarität dem Wasser nachgeordnete Lösungsmittel bzw. Polyoxymethylene, - ethylene oder Emulgartoren wie Glycolipide, Partialglyceride bzw. Phosphatide zuzusetzen. Den wirksamen Basisprodukten können ansonsten in der Kosmetik übliche Ingredienzen zugesetzt werden.

## Patentansprüche

1. Hyaferm-Komposit-Gele als topisch wirksame Basisprodukte, **dadurch gekennzeichnet, daß** diese neben Wasser oder anteilig polaren und mit Wasser mischbaren Flüssigkeiten Hyaferm in einer Konzentration von 0,1-2,5 Gewichtsprozent, vornehmlich 0,5-1%, und Polysaccharide mit β-anomeren Saccharideinheiten wie Xylo-, Sclero-, Hefe- und Pilzglucanen sowie Arabinoxylanen und Licheninen mit einer Konzentration von 0,001-0,5 Gewichtsprozent, vornehmlich 0,005-0,1% enthalten.
